# EUROPEAN PATENT APPLICATION

(11) **EP 4 775 222 A1**
(43) Date of publication of application: **15.07.2026**
(21) Application number: 24862749.9
(22) Date of filing: 02.09.2024
(51) Int. Cl.: A61K 39/12, A61K 35/76, A61K 38/20, A61P 31/14, A61P 37/04, A61P 43/00, C12N 7/01, C12N 15/24, C12N 15/40, C12N 15/86, C12N 15/863

(54) **DENGUE VIRUS VACCINE AND DENGUE VIRUS VACCINE KIT**

(30) Priority: 07.09.2023 JP 2023144930
(71) Applicant: Kagoshima University, Kagoshima-shi, Kagoshima 890-8580 (JP); NAGASAKI UNIVERSITY, Nagasaki 852-8521 (JP); Kohara, Michinori, Tokyo 156-8506 (JP)
(72) Inventor: KOHARA Kyoko, Kagoshima-shi, Kagoshima 890-8580 (JP); MORITA Kouichi, Nagasaki-shi, Nagasaki 852-8521 (JP); YASUTOMI Yasuhiro, Tsukuba-shi, Ibaraki 305-0843 (JP); ISHII Koji, Tokyo 162-8640 (JP); KOHARA Michinori, Tokyo 156-8506 (JP)
(74) Representative: v. Bezold & Partner Patentanwälte - PartG mbB
(86) International application number: PCT/JP2024/031425
(87) International publication number: WO 2025/053097

(57) **Abstract**

A dengue virus vaccine includes a recombinant vaccinia virus having a DNA encoding a region other than NS1 in non-structural proteins of a dengue virus and having a promoter controlling expression of the region other than NS1, wherein the vaccine is inoculated into an inoculation subject two or more times.

## Description

### Technical Field

The present disclosure relates to a dengue virus vaccine and a dengue virus vaccine kit.

### Background Art

Dengue virus (hereinafter also referred to as "DENV") is said to be endemic mainly in tropical regions, infects approximately 400 million people annually in the world, and causes dengue fever in approximately 100 million people. In Japan, the 2014 outbreak has ended. However, because DENV is brought in every year via travelers and the like from infection endemic countries, Japan is in danger of chronic domestic outbreaks. The number of annual imported cases reported since 2010 exceeds 200 cases.

Currently, there is no therapeutic drug usable clinically, and symptomatic treatment is performed. On the other hand, clinical trials of vaccines against DENV are proceeding. Sanofi Pasteur's chimeric yellow fever-dengue tetravalent dengue vaccine (CYD-TDV) that is the most developed was approved for the first time in Mexico in December, 2015, and has been approved in 20 countries. However, there is a report that inoculating CYD-TDV into non-infected persons increases the onset risk of dengue fever and dengue hemorrhagic fever (see Non-Patent Literature 1).

DENV belongs to the Flaviviridae family, and four serotypes (Serotype-1, 2, 3, and 4) of DENV exist. Infection with DENV, when followed by infection with DENV belonging to a different serotype, causes a phenomenon called antibody-dependent enhancement (ADE) in which an antibody against the first DENV helps infection with the second DENV. Through this phenomenon, excessive production of DENV is caused, leading to the onset of a severe disease such as dengue hemorrhagic fever.

An antibody against a structural protein may induce ADE. To avoid ADE, Patent Literature 1 discloses a recombinant vaccinia virus having a gene encoding NS1 or a region other than NS1 in non-structural proteins of DENV.

### Citation List

### Patent Literature

Patent Literature 1: Unexamined Japanese Patent Application Publication No. 2020-150940

### Non Patent Literature

Non Patent Literature 1: The World Health Organization (WHO), "Dengue vaccine safety update", 2018 (URL:https://www.who.int/groups/global-advisory-committee-on-vaccine-safety/topics/dengue-vaccines/safety-update)

### Summary of Invention

### Technical Problem

The recombinant vaccinia virus disclosed in Patent Literature 1 is useful as a vaccine that does not induce ADE. There is a need to further enhance the antiviral effect through examining the usage of the recombinant vaccinia virus.

The present disclosure has been made in view of the above circumstances, and an object of the present disclosure is to provide a dengue virus vaccine and a dengue virus vaccine kit having a high antiviral effect.

### Strategy to solve subjects

### (Dengue Virus Vaccine)

A dengue virus vaccine according to the present disclosure includes
a recombinant vaccinia virus having a DNA encoding a region other than NS1 in non-structural proteins of a dengue virus, and having a promoter controlling expression of the region other than NS1,
wherein the vaccine is inoculated into an inoculation subject two or more times.

The dengue virus vaccine according to the present disclosure includes
a recombinant vaccinia virus having a DNA encoding a region other than NS1 in non-structural proteins of a dengue virus, and having a promoter controlling expression of the region other than NS1,
wherein the vaccine is used in combination with a viral vector expressing IL-15.

The dengue virus vaccine according to the present disclosure includes
a recombinant vaccinia virus having a DNA encoding a region other than NS1 in non-structural proteins of a dengue virus, and having a promoter controlling expression of the region other than NS1; and
a viral vector expressing IL-15.

The dengue virus vaccine according to the present disclosure includes
a recombinant vaccinia virus having a DNA encoding a region other than NS1 in non-structural proteins of a dengue virus, and having a promoter controlling expression of the region other than NS1,
wherein the vaccine is inoculated into an inoculation subject to which an expression vector expressing the region other than NS1 in the non-structural proteins of a dengue virus has been administered.

### (Dengue Virus Vaccine Kit)

A dengue virus vaccine kit according to the present disclosure includes
a recombinant vaccinia virus having a DNA encoding a region other than NS1 in non-structural proteins of a dengue virus, and having a promoter controlling expression of the region other than NS1; and
a viral vector expressing IL-15.

The dengue virus vaccine kit according to the present disclosure includes
a recombinant vaccinia virus having a DNA encoding a region other than NS1 in non-structural proteins of a dengue virus, and having a promoter controlling expression of the region other than NS1; and
an expression vector expressing the region other than NS1 in the non-structural proteins of a dengue virus.

The region other than NS1 may be a region consisting of NS2A, NS2B, NS3, NS4A, NS4B and NS5.

A serotype of the dengue virus may be type 2.

A gene encoding the NS5 in the DNA may have a mutation that reduces RNA polymerase activity of the NS5.

The gene encoding the NS5 in the DNA may encode the amino acid sequence of the following (a) or (b):
(a) an amino acid sequence of SEQ ID NO: 1; or
(b) an amino acid sequence having a sequence identity of 90% or more to the amino acid sequence of SEQ ID NO: 1.

### Solution to Problem

### Advantageous Effects of Invention

The dengue virus vaccine and the dengue virus vaccine kit according to the present disclosure have a high antiviral effect.

### Brief Description of Drawings

FIG. 1 is a diagram schematically illustrating the structure of the genome of DENV.
FIG. 2 is a diagram illustrating a change over time in the copy number of DENV type 2 RNA present in 1 mL of blood in Test Example.
FIG. 3 is a diagram illustrating a change over time in the copy number of DENV type 2 RNA present in 1 mL of blood in Test Example.
FIG. 4A is a diagram illustrating the results of ELISpot of splenic cells cultured in the presence of NS3 peptide in Test Example.
FIG. 4B is a diagram illustrating the results of ELISpot of splenic cells cultured in the presence of NS4A peptide in Test Example.

### Description of Embodiments

Embodiments according to the present disclosure are described with reference to the drawings. Note that the present disclosure is not limited by the following embodiments and drawings. In the following embodiments, expressions such as "having", "including", or "containing" also encompass the meaning of "consisting of" or "constituted by".

### [DENV Vaccine]

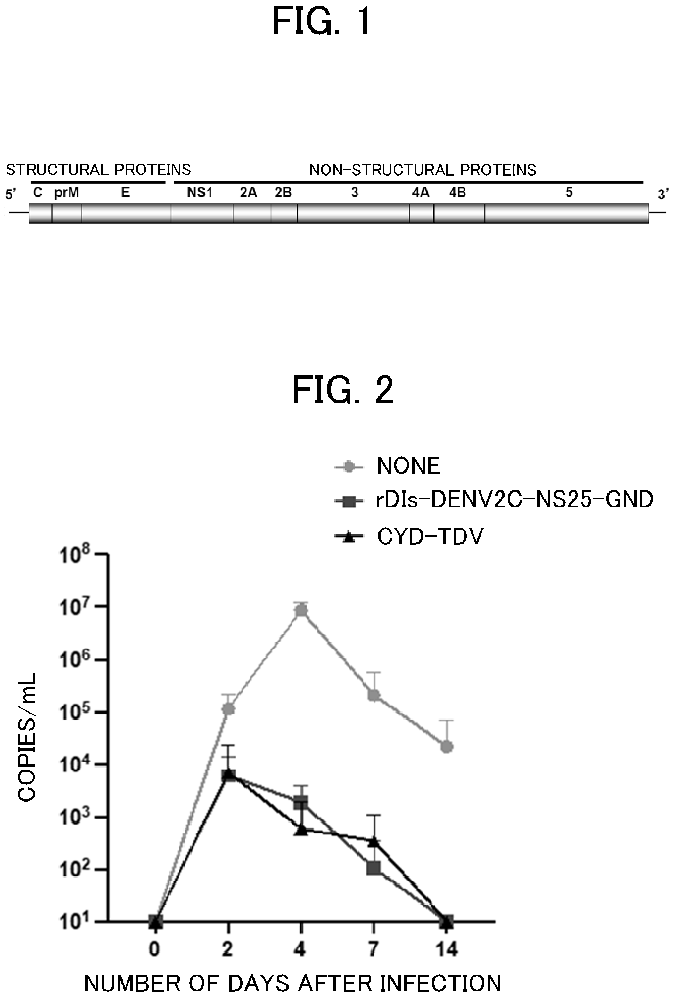

The genome of DENV is single-stranded RNA. As illustrated in FIG. 1, the genome of DENV encodes three types of structural proteins and seven types of non-structural proteins between a 5' terminal noncoding region and a 3' terminal noncoding region. The structural proteins are C, prM, and E. The non-structural proteins are NS1, NS2A, NS2B, NS3, NS4A, NS4B, and NS5. The DENV vaccine according to the present embodiment includes a recombinant vaccinia virus having a DNA encoding a region other than NS1 in non-structural proteins of DENV, and having a promoter controlling expression of the DNA, that is, expression of the region other than NS1. "Encoding a region other than NS1 in non-structural proteins of DENV" means encoding a region other than NS1, the region produced in a cell when the virus proliferates. More specifically, the region other than NS1 in the non-structural proteins is a region consisting of NS2A, NS2B, NS3, NS4A, NS4B, and NS5 (hereinafter, collectively referred to as "NS2-5 region").

The serotype of DENV from which the DNA encoding the region other than NS1 in the non-structural proteins of DENV according to the present embodiment is derived is not particularly limited, but is preferably type 2. The type 2 is further divided into an Asian type and a Cosmopolitan strain, and as the DNA encoding the region other than NS1, a DNA derived from the Cosmopolitan strain of the type 2 is particularly preferable. For example, when the gene encoding the NS2-5 region is derived from the Cosmopolitan strain of the type 2, the nucleotide sequence of the DNA is shown in, for example, SEQ ID NO: 2.

The DNA encoding the region other than NS1 in the non-structural proteins of DENV is obtained by a general genetic engineering technique. For example, as the genetic engineering technique, a nucleic acid synthesis method using a generally used DNA synthesizer may be used. A method that can be alternatively used is: a PCR method in which isolating a nucleotide sequence of a gene serving as a template is followed by designing primers specific to respective genes, and amplifying the nucleotide sequence using a PCR device; or a gene amplification method using a cloning vector. Preferably, the DNA encoding the region other than NS1 in the non-structural proteins can be obtained by performing PCR using a DENV gene introduced into a plasmid as a template and using primers specific to a sequence encoding the region in the DENV gene, the region being other than NS1 in the non-structural proteins.

In order not to suppress proliferation of the recombinant vaccinia virus, a gene encoding NS5 in the DNA may have a mutation that reduces RNA polymerase activity of NS5. For reduction of the RNA polymerase activity, the RNA polymerase activity of the encoded NS5 may be lower than the RNA polymerase activity of wild-type NS5, and the RNA polymerase activity of NS5 is preferably inactivated. In this case, the gene encoding NS5 in the DNA encodes, for example, the following amino acid sequence (a) or (b):
(a) an amino acid sequence of SEQ ID NO: 1; or
(b) an amino acid sequence having a sequence identity of 90% or more to the amino acid sequence of SEQ ID NO: 1.

In the present embodiment, "sequence identity" refers to a ratio of the number of sites where the same amino acid exists in both of two amino acid sequences to the number of amino acids in the full length when the amino acid sequences are aligned so that the degree of match is maximized.

"Sequence identity of 90% or more" regarding the amino acid sequence means a sequence identity of at least 90%. The amino acid sequence of NS5 having a mutation may have a sequence identity of 90% or more, preferably 92% or more, more preferably 95% or more, still more preferably 97% or more, even more preferably 98% or more, and particularly preferably 99% or more to the amino acid sequence of SEQ ID NO: 1.

When the gene encoding NS5 in the DNA has a mutation that reduces the RNA polymerase activity of NS5, the nucleotide sequence of the DNA is shown in, for example, SEQ ID NO: 3.

The promoter controlling expression of the region other than NS1 is not particularly limited. As the expression promoter contained in the vaccinia virus, various expression promoters used for gene expression of the vaccinia virus can be used. Examples of the promoter include Pm2H5, PsynII, mH5 promoters, and the like.

In the production of the recombinant vaccinia virus according to the present embodiment, although not particularly limited, for example, the DNA encoding the region other than NS1 in the non-structural proteins of DENV and the promoter controlling expression of the region other than NS1 may be introduced into a vector (plasmid), and the vector may be introduced into the vaccinia virus. As the vector, for example, a pSMART (trademark) vector or the like can be used.

Although not particularly limited, the vaccinia virus is preferably an attenuated strain. As the vaccinia virus, for example, a DIs strain isolated through passaging a vaccinia virus Dalian strain (DIE strain) in chicken embryos is preferable. The DIs strain proliferates in primary chicken fibroblast cells but hardly proliferates in other mammalian cells. Accordingly, safety is ensured even if the recombinant vaccinia virus is inoculated into an immunodeficient or immunosuppressed subject.

As introduction of the vector into the vaccinia virus, any known method can be adopted. For example, introducing the above-mentioned vector into an animal cell infected with the vaccinia virus causes homologous recombination in the genome of the vaccinia virus, and thus, allows production of the recombinant vaccinia virus expressing the region other than NS1 in the non-structural proteins of DENV.

In order to confirm introduction of the DNA encoding the region other than NS1 in the non-structural proteins of DENV into the recombinant vaccinia virus, PCR may be performed using the genome of the recombinant vaccinia virus as a template and using primers specific to the DNA. In addition, expression of the region other than NS1 in the non-structural proteins of DENV can be confirmed by Western blotting using, as a sample, an animal cell infected with the recombinant vaccinia virus. Note that as an antibody in Western blotting, for example, a commercially available antibody specifically recognizing a region other than NS1, or IgG purified by Protein G from an antiserum produced by immunizing a DENV polypeptide can be used.

The DENV vaccine according to the present embodiment can be used as a prophylactic agent and a therapeutic agent for a DENV infection. Examples of the DENY infection include dengue fever, dengue hemorrhagic fever, and the like.

The DENV vaccine according to the present embodiment may be inoculated parenterally or may be inoculated orally. Depending on the mode of inoculation, the DENV vaccine may be made into tablets, capsules, powders, granules, pills, liquids, syrups, injections, external preparations, suppositories, eye drops, or the like. The mode of inoculation of the DENV vaccine is preferably local injection into a subcutaneous part, intradermal part, muscle, intraperitoneal part, or the like. In addition, the DENV vaccine may be inoculated into an inoculation subject by inhalation from, for example, the nasal cavity, oral cavity, or lungs. Preferably, to stimulate subcutaneous immune cells, the DENV vaccine is inoculated subcutaneously into the inoculation subject. For subcutaneous inoculation, a scarification method may be used, or a non-invasive method such as a method using a microneedle may be adopted.

The DENV vaccine according to the present embodiment may contain, in addition to the recombinant vaccinia virus as an active ingredient, a pharmaceutically acceptable carrier such as an excipient, bulking agent, binder, or lubricant, and/or a buffer, isotonizing agent, chelating agent, colorant, preservative, fragrance, flavoring agent, sweetener, and/or the like.

Inoculation subjects of the DENV vaccine according to the present embodiment are humans and animals other than humans, and preferably mammals. Examples of the inoculation subjects include humans; primates such as chimpanzees; experimental animals such as rats, mice, and rabbits; livestock animals such as pigs, cows, horses, sheep, and goats; and pet animals such as dogs and cats.

The dosage in inoculation of the DENV vaccine according to the present embodiment is suitably set depending on the inoculation route, the inoculation subject, and the age, body weight, sex, symptom, and other conditions of the inoculation subject. The daily dosage of the recombinant vaccinia virus is, for example, 1,000 to 1,000,000,000 PFU (plaque forming units), preferably 100,000 to 100,000,000 PFU in the case of oral administration, and for example, 100 to 1,000,000,000 PFU, preferably 1,000 to 100,000,000 PFU in the case of parenteral administration but is not limited to these ranges.

The DENV vaccine according to the present embodiment is preferably inoculated into an inoculation subject two or more times. The number of inoculations is not particularly limited as long as the inoculations induce cellular immunity sufficiently. The number of inoculations is, for example, two or three times. The dosage of the DENV vaccine in the first inoculation and the dosage of the DENV vaccine in the second or subsequent inoculation into the same inoculation subject may be the same or different. The interval of inoculation into the same inoculation subject may be suitably set according to the situation of induction of cellular immunity, and the second inoculation is, for example, after a lapse of 1 day, 3 days, 5 days, 7 days, 10 days, 2 weeks, 3 weeks, 4 weeks, 6 weeks, or 2 months from the first inoculation. The interval between the second inoculation and the third inoculation into the same inoculation subject is the same as the interval between the first inoculation and the second inoculation. The inoculation interval between the first time and the second time and the inoculation interval between the second time and the third time may be the same or different. Furthermore, the second or subsequent inoculation may be by the same route as the first inoculation or may be by a different route.

Furthermore, the recombinant vaccinia virus according to the present embodiment may be used in combination with a viral vector expressing interleukin 15 (IL-15). By using the recombinant vaccinia virus and IL-15 in combination, highly active DENV-specific CD8⁺ T cells can be induced and maintained for a long period of time. The viral vector is not particularly limited, and examples of the viral vector include retroviruses, lentiviruses, adenoviruses, adeno-associated viruses, herpes simplex viruses, and the like. As the viral vector, a vaccinia virus vector can be preferably used. The viral vector has a DNA encoding IL-15 and a promoter controlling expression of IL-15. A method of producing the viral vector is the same as the above-described method of producing the recombinant vaccinia virus. The DNA encoding the region other than NS1 in the non-structural proteins of DENV in the above-described method of producing the recombinant vaccinia virus can be referred to with the DENV DNA replaced with the DNA encoding IL-15.

The mode of combined use of the recombinant vaccinia virus according to the present embodiment and the viral vector expressing IL-15 is not particularly limited, and the viral vector may be inoculated into the inoculation subject of the recombinant vaccinia virus. The recombinant vaccinia virus and the viral vector may be inoculated simultaneously, or one may be inoculated and then the other may be inoculated after a lapse of a certain period. The dosage in inoculation of the viral vector is suitably set depending on the inoculation route, the inoculation subject, and the age, body weight, sex, symptoms, and other conditions of the inoculation subject. The daily dosage of the viral vector is, for example, 1,000 to 1,000,000,000 PFU, preferably 100,000 to 100,000,000 PFU in the case of oral administration, and for example, 100 to 1,000,000,000 PFU, preferably 1,000 to 100,000,000 PFU in the case of parenteral administration but is not limited thereto.

Additionally, the recombinant vaccinia virus according to the present embodiment may be inoculated into an inoculation subject to which an expression vector expressing a region other than NS1 in the non-structural proteins of DENV has been administered. The expression vector is not particularly limited, and a known expression vector can be used. As the expression vector, a commercially available expression vector may be used. Examples of the expression vector include a pCAGGS-Neo viral vector. In the production of the expression vector, the DNA encoding the region other than NS1 in the non-structural proteins of DENV may be amplified by PCR, and the obtained PCR product may be introduced into a position under expression control by a promoter in the expression vector.

The expression vector may be administered as a pharmaceutical composition containing a pharmaceutically acceptable carrier such as an excipient, bulking agent, binder, or lubricant, and/or a buffer, isotonizing agent, chelating agent, colorant, preservative, fragrance, flavoring agent, sweetener, and/or the like.

The mode of administration of the expression vector is not particularly limited, and the expression vector may be administered parenterally or may be administered orally. Depending on the mode of administration, the expression vector can be made into tablets, capsules, powders, granules, pills, liquids, syrups, injections, external preparations, suppositories, eye drops, or the like. The mode of administration of the expression vector is preferably local injection into a subcutaneous part, intradermal part, muscle, intraperitoneal part, or the like. In addition, the expression vector may be administered to the inoculation subject by inhalation from, for example, the nasal cavity, oral cavity, or lungs. Preferably, the expression vector is administered subcutaneously to the inoculation subject.

The interval of inoculation of the recombinant vaccinia virus according to the present embodiment after administration of the expression vector may be suitably set according to the situation of induction of cellular immunity. For example, the recombinant vaccinia virus may be inoculated after a lapse of 1 day, 3 days, 5 days, 7 days, 10 days, 2 weeks, 3 weeks, 4 weeks, 6 weeks, or 2 months from administration of the expression vector. The dosage of the expression vector is suitably set depending on the inoculation route, the inoculation subject, and the age, body weight, sex, symptoms, and other conditions of the inoculation subject.

By inoculating the DENV vaccine according to the present embodiment two or more times, the DENV vaccine exhibits an antiviral effect at least equivalent to the antiviral effect of CYD-TDV as described in Examples below. In addition, by using the DENV vaccine in combination with the expression vector, the DENV vaccine exhibits an antiviral effect at least equivalent to the antiviral effect of CYD-TDV even with a single inoculation. Furthermore, by using the DENV vaccine in combination with the viral vector, a remarkable antiviral effect can be obtained.

The DENV vaccine according to the present embodiment has an antiviral effect and an onset protective effect by immune response not only against DENV of the serotype from which the DNA encoding the region other than NS1 is derived but also against DENV of a serotype different from the serotype. The DENV vaccine according to the present embodiment can suppress induction of pathology of a severe disease, the induction being due to ADE, and particularly problematic in DENV infection.

In another aspect of the present embodiment, provided is a DENV vaccine including: a recombinant vaccinia virus having a DNA encoding a region other than NS1 in non-structural proteins of DENV, and having a promoter controlling expression of the DNA; and a viral vector expressing IL-15.

In another aspect of the present embodiment, a DENV vaccine kit is provided. The DENV vaccine kit includes: a recombinant vaccinia virus having a DNA encoding a region other than NS1 in non-structural proteins of DENV and having a promoter controlling expression of the DNA; and a viral vector expressing IL-15. Furthermore, in another aspect of the present embodiment, provided is a DENV vaccine kit including: a recombinant vaccinia virus having a DNA encoding a region other than NS1 in non-structural proteins of DENV, and having a promoter controlling expression of the DNA; and an expression vector expressing the region other than NS1 in the non-structural proteins of DENV. The DENV vaccine kit includes the viral vector and the expression vector in a mode separated from the recombinant vaccinia virus, respectively. For example, in the DENV vaccine kit, the recombinant vaccinia virus is held in a first container, and the viral vector or the expression vector is held in a second container different from the first container. In this case, a user mixes the recombinant vaccinia virus held in the first container and the viral vector or the expression vector held in the second container at the time of use, whereby the DENV vaccine can be prepared at the time of use.

The present disclosure is described more specifically with reference to the following Examples, but the present disclosure is not limited by the Examples.

### Examples

### Example 1: Production of DENV non-structural protein recombinant vaccinia vaccine

A vaccine was created based on the genome of DENV type 2 Cosmopolitan strain (Dengue-2 Cosmopolitan: o1Sa-054, hereinafter referred to as "DENV2"). The nucleotide sequence of the gene encoding the NS2-5 region of the DENV type 2 Cosmopolitan strain is shown in SEQ ID NO: 2.

The gene of the NS2-5 region was amplified by PCR using cDNA encoding the non-structural proteins of DENV2 as a template and using NS2-5 region primers (a forward primer whose nucleotide sequence is shown in SEQ ID NO: 4 and a reverse primer whose nucleotide sequence is shown in SEQ ID NO: 5). The obtained PCR product was digested with Sbf-I enzyme and AsiSI enzyme, and then introduced into a transfer vector pUC/DIs (Koji Ishii et. al, Virology, 2001, 302, 433-444) that is a DIs strain recombinant plasmid. The obtained plasmid is referred to as "DENV 2C NS25 plasmid".

The reaction solution composition and reaction conditions of PCR using the NS2-5 region primers are as follows.

### [Reaction solution composition]

Template cDNA (DENV 2C cDNA): 1.0 µL
5×Q5 DNA polymerase buffer: 5 µL
2.5 mM dNTP: 2.5 µL
Q5 DNA polymerase (2.0 U/µl): 0.5 µL
Forward primer (10 µM): 1.25 µL
Reverse primer (10 µM): 1.25 µL
Sterilized water: 13.5 µL
Total: 25 µL

### [Reaction conditions]

A total of 35 cycles, with each cycle including "thermal denaturation and dissociation: 98°C for 10 seconds -> annealing: 72°C for 15 seconds -> synthesis and extension: 72°C for 40 seconds"

Subsequently, to reduce the RNA polymerase activity of NS5, the gene encoding NS5 was modified by PCR using QuickChange (trademark) Lightning Site-Directed Mutagenesis kit (manufactured by Stratagene, #210518) so that the amino acid sequence "GDD" in NS5 became "GND". For the PCR, NS2-5 region mutation introduction primers (a forward primer whose nucleotide sequence is shown in SEQ ID NO: 6 and a reverse primer whose nucleotide sequence is shown in SEQ ID NO: 7) were used. The reaction solution composition and reaction conditions of PCR using the NS2-5 region mutation introduction primers are as follows.

### [Reaction solution composition]

Template DNA (DENV 2C NS25 plasmid): 1.0 µL
10×reaction buffer: 2.0 µL
2.5 mM dNTP: 0.4 µL
F primer (50 ng/ul): 1.0 µL
R primer (50 ng/ul): 1.0 µL
QuickSolution reagent: 0.6 µL
QuickChange Lightning Enzyme: 0.4 µL
Sterilized water: 13.6 µL
Total: 20 µL

### [Reaction conditions]

A total of 18 cycles, with each cycle including "thermal denaturation and dissociation: 95°C for 20 seconds -> annealing: 60°C for 10 seconds -> synthesis and extension: 68°C for 6 minutes"

The obtained PCR product was introduced into the DIs strain recombinant plasmid in the same manner as the PCR product in PCR with the above-mentioned NS2-5 region primers. Then, the DIs strain recombinant plasmid was introduced downstream of the mH5 promoter of pSMART-DIs-L3-GPTF (manufactured by GenScript Biotech Corporation), a recombinant vector for producing a DIs strain recombinant vaccine. The recombinant vector has the E. coli gpt gene under the control of VACV p7.5, an early promoter.

The obtained recombinant vector was linearized by ApaI restriction enzyme digestion. The linearized vector was purified and introduced into primary chicken embryo fibroblast cells that had previously been infected with the attenuated vaccinia virus DIs strain at a multiplicity of infection of 10 for 1 hour. Twenty hours after introduction, the cell layer was detached to collect a mixture containing the virus and the cells, and the obtained suspension was frozen at -80°C until use.

A recombinant vaccinia virus (rDIs-DENV2C-NS25-GND) expressing the NS2-5 region having a mutation in NS5 resulted from homologous recombination caused in the genome of the DIs strain by introduction of the vector. The virus was purified using mycophenolic acid (a purine metabolism inhibitor) as a selection reagent. rDIs-DENV2C-NS25-GND having the E. coli gpt gene can be cultured even if the GMP synthetic route is inhibited by mycophenolic acid in the presence of xanthine and hypoxanthine.

### Example 2: Production of IL-15 recombinant vaccinia vaccine

A DNA (SEQ ID NO: 8) having an mH5 promoter and an IL-15 gene between Hind III recognition sites was artificially synthesized, and introduced into a DIs strain recombinant plasmid using Hind III. Furthermore, the DIs strain recombinant plasmid was introduced into pSMART-DIs-L3-GPTF (manufactured by GenScript), and a recombinant vaccinia virus (rDIs-IL-15) expressing IL-15 was produced in the same manner as the above rDIs-DENV2C-NS25-GND.

### Example 3: Production of DENV non-structural protein gene expression vector

A vector (pCAG-NS25) expressing the NS2-5 region having a mutation in NS5 was prepared by introducing the PCR product obtained by PCR using the NS2-5 region mutation introduction primers in Example 1 into a pCAGGS-Neo vector.

### Test Example: DENV infection experiment

A 1/10 of the human dose of dengue fever vaccine CYD-TDV (manufactured by Sanofi Pasteur S.A.) or 1 × 10⁸ PFU of rDIs-DENV2C-NS25-GND was subcutaneously inoculated into A129 mice (10 weeks old, manufactured by Marshall BioResources) by a scarification method. Four weeks later, the same vaccine as in the first inoculation was inoculated in the second inoculation into each individual in the same manner as in the first inoculation. Furthermore, four weeks later, the mice were intraperitoneally infected with DENV2 (10⁴ PFU/mouse). Blood was collected from the mice on day 0, day 2, day 4, day 7, day 11, and day 14 after infection. Note that the mice in the control group (None) were infected with DENV2 without being inoculated with a vaccine.

In addition, the DENV infection experiment was similarly performed by changing the number of inoculations or the type of the vaccine to be inoculated, as shown in Table 1. Blood was collected from the mice on day 0, day 2, day 4, and day 7 after infection. Note that, into Group D, a vaccine mixture of 1 × 10⁸ PFU of rDIs-DENV2C-NS25-GND and 1 × 10⁸ PFU of rDIs-IL-15 was inoculated each time. Regarding pCAG-NS25, 100 µg was administered using Actranza (trademark) Lab (manufactured by Daicel Corporation).

**[Table 1]**

| Group | First inoculation | Second inoculation |
|---|---|---|
| A | rDIs-DENV2C-NS25-GND | None |
| B | pCAG-NS25 | rDIs-DENV2C-NS25-GND |
| C | rDIs-DENV2C-NS25-GND | rDIs-DENV2C-NS25-GND |
| D | rDIs-DENV2C-NS25-GND and DIs-IL-15 | rDIs-DENV2C-NS25-GND and rDIs-IL-15 |

DENV2 in the blood was quantified by quantitative reverse transcription PCR (qRT-PCR). RNA was extracted from the blood using Isogen (manufactured by Nippon Gene Co., Ltd.), and RNA of DENV2 was quantified with a sense primer whose nucleotide sequence is shown in SEQ ID NO: 9, an antisense primer whose nucleotide sequence is shown in SEQ ID NO: 10, and a probe whose nucleotide sequence is shown in SEQ ID NO: 11 (all manufactured by Thermo Fisher Scientific Inc.). Note that the 5' terminal of the probe was labeled with FAM.

The degree of activation of cellular immunity was evaluated by an ELISpot assay using single splenic cells isolated according to a conventional method from spleens collected from the mice of the control group and Groups A to D 14 days after infection. In each well of an ELISpot plate coated with an anti-mouse IFN-γ antibody (#3321-2H, manufactured by Mabtech AB), the splenic cells were cultured for 24 hours in a medium containing 1 µg/mL of NS3 peptide or NS4A peptide of DENV according to the manufacturer's instructions. The composition of the medium was RPMI1640 (manufactured by Shimadzu Diagnostics Corporation) containing 10% fetal bovine serum, L-glutamine (4 mM), penicillin-streptomycin (100 U/mL), HEPES (10 mM), 2-mercaptoethanol (50 µM), non-essential amino acids, and 0.2% sodium carbonate. After drying the ELISpot plate, the number of spots in each well was counted with an automatic ELISpot plate reader (manufactured by Advanced Imaging Devices GmbH).

### (Results)

FIG. 2 illustrates the copy number of RNA of DENV2 present in 1 mL of blood after infection. By inoculating rDIs-DENV2C-NS25-GND twice, an antiviral effect equivalent to the antiviral effect of CYD-TDV was obtained.

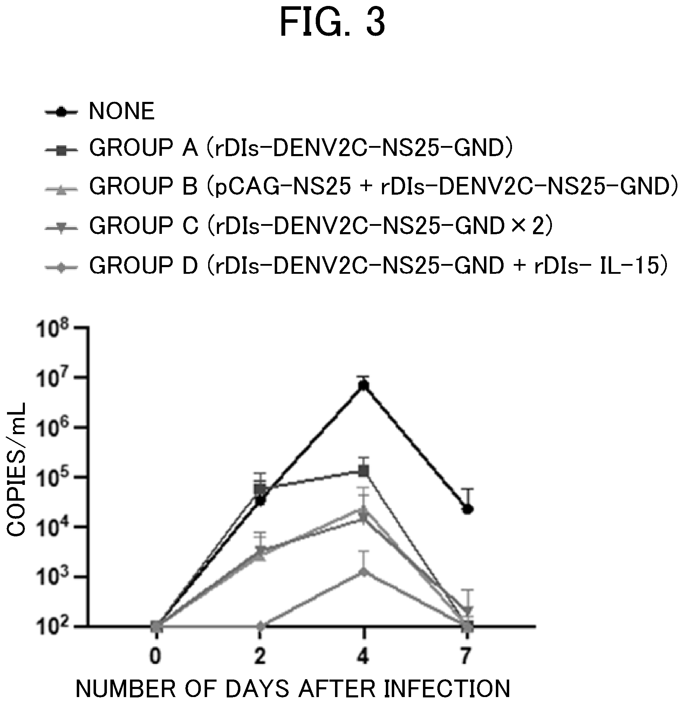

Regarding Groups A to D, the copy number of RNA of DENV2 present in 1 mL of blood after infection is illustrated in FIG. 3. By inoculating rDIs-DENV2C-NS25-GND after immunization with pCAG-NS25 in advance (Group B), an antiviral effect equivalent to the antiviral effect in the case where rDIs-DENV2C-NS25-GND was inoculated twice (Group C) was obtained. Inoculating a mixture of rDIs-DENV2C-NS25-GND and rDIs-IL-15 (Group D) has revealed that DENV in the blood can be suppressed to approximately 1/10,000 compared to the control group (None).

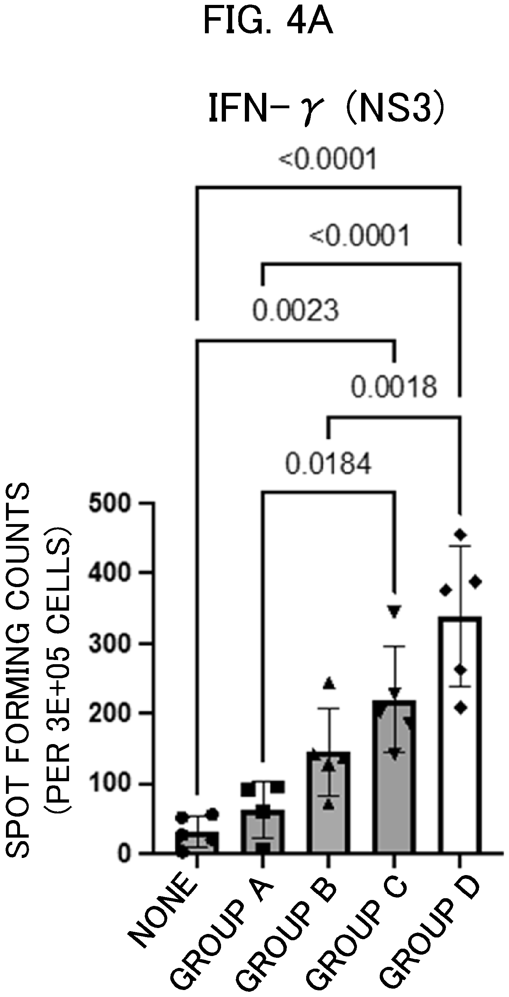

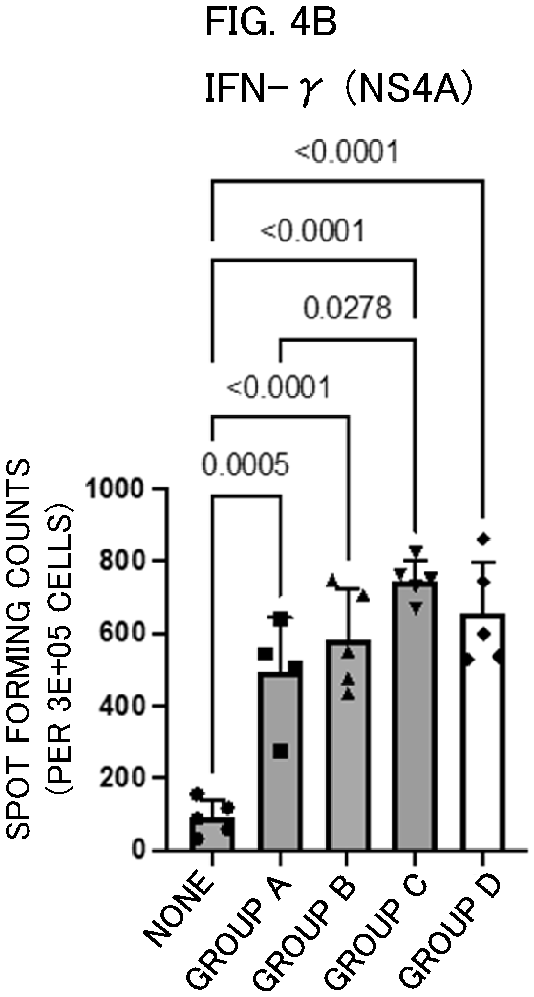

The results of ELISpot of the splenic cells cultured in the presence of NS3 peptide are illustrated in FIG. 4A. The results of ELISpot of the splenic cells cultured in the presence of NS4A peptide are illustrated in FIG. 4B. Regardless of whether the Groups were stimulated with NS3 peptide or NS4A peptide, the amount of secretion of IFN-γ increased relative to the control group, indicating that cellular immunity was activated. Remarkable activation was seen in Group D stimulated with NS3 peptide.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-144930, filed on September 7, 2023, the entire disclosure of which is incorporated by reference herein.

### Industrial Applicability

The present disclosure is useful for a vaccine against DENV.

## Claims

1. A dengue virus vaccine comprising a recombinant vaccinia virus having a DNA encoding a region other than NS1 in non-structural proteins of a dengue virus, and having a promoter controlling expression of the region other than NS1,
wherein the vaccine is inoculated into an inoculation subject two or more times.

2. A dengue virus vaccine comprising a recombinant vaccinia virus having a DNA encoding a region other than NS1 in non-structural proteins of a dengue virus, and having a promoter controlling expression of the region other than NS1,
wherein the vaccine is used in combination with a viral vector expressing IL-15.

3. A dengue virus vaccine comprising:
a recombinant vaccinia virus having a DNA encoding a region other than NS1 in non-structural proteins of a dengue virus, and having a promoter controlling expression of the region other than NS1; and
a viral vector expressing IL-15.

4. A dengue virus vaccine comprising a recombinant vaccinia virus having a DNA encoding a region other than NS1 in non-structural proteins of a dengue virus, and having a promoter controlling expression of the region other than NS1,
wherein the vaccine is inoculated into an inoculation subject to which an expression vector expressing the region other than NS1 in the non-structural proteins of a dengue virus has been administered.

5. A dengue virus vaccine kit comprising:
a recombinant vaccinia virus having a DNA encoding a region other than NS1 in non-structural proteins of a dengue virus, and having a promoter controlling expression of the region other than NS1; and
a viral vector expressing IL-15.

6. A dengue virus vaccine kit comprising:
a recombinant vaccinia virus having a DNA encoding a region other than NS1 in non-structural proteins of a dengue virus, and having a promoter controlling expression of the region other than NS1; and
an expression vector expressing the region other than NS1 in the non-structural proteins of a dengue virus.

7. The dengue virus vaccine according to claim 1, 2, 3 or 4, or the dengue virus vaccine kit according to claim 5 or 6,
wherein the region other than NS1 is a region consisting of NS2A, NS2B, NS3, NS4A, NS4B and NS5.

8. The dengue virus vaccine according to claim 1, 2, 3 or 4, or the dengue virus vaccine kit according to claim 5 or 6,
wherein a serotype of the dengue virus is type 2.

9. The dengue virus vaccine or dengue virus vaccine kit according to claim 7,
wherein a gene encoding the NS5 in the DNA has a mutation that reduces RNA polymerase activity of the NS5.

10. The dengue virus vaccine or dengue virus vaccine kit according to claim 9,
wherein the gene encoding the NS5 in the DNA encodes the amino acid sequence of the following (a) or (b):
(a) an amino acid sequence of SEQ ID NO: 1; or
(b) an amino acid sequence having a sequence identity of 90% or more to the amino acid sequence of SEQ ID NO: 1.
